(19) Europäisches Patentamt
European Patent Office
Office européen des brevets

(11) Publication number: **0 149 324**
**B1**

(12) # EUROPEAN PATENT SPECIFICATION

(45) Date of publication of patent specification: **02.03.88**

(21) Application number: **84308335.3**

(22) Date of filing: **30.11.84**

(51) Int. Cl.[4]: **C 07 C 103/76,**
C 07 C 121/457,
C 07 C 153/063,
C 07 D 213/82, A 01 N 37/46,
A 01 N 43/40

(54) **Biologically active amide derivatives.**

(30) Priority: **19.01.84 GB 8401410**
**22.02.84 GB 8404702**

(43) Date of publication of application:
**24.07.85 Bulletin 85/30**

(45) Publication of the grant of the patent:
**02.03.88 Bulletin 88/09**

(84) Designated Contracting States:
**AT BE CH DE FR GB IT LI NL**

(56) References cited:
**EP-A-0 059 536**
**EP-A-0 076 030**

(73) Proprietor: **IMPERIAL CHEMICAL INDUSTRIES PLC**
**Imperial Chemical House Millbank**
**London SW1P 3JF (GB)**

(72) Inventor: **Crowley, Patrick Jelf**
**56 Ellis Road**
**Crowthorne Berkshire (GB)**
Inventor: **Shephard, Margaret Claire**
**14 Lambourne Drive**
**Maidenhead Berkshire (GB)**

(74) Representative: **Alner, Henry Giveen Hamilton et al**
**Imperial Chemical Industries PLC Legal Department: Patents P.O. Box 6 Bessemer Road**
**Welwyn Garden City Herts, AL7 1HD (GB)**

**EP 0 149 324 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to substituted benzamide derivatives useful as herbicides and fungicides, to processes of combatting weeds and fungal infestations and to herbicidal and fungicidal compositions.

Substituted benzamide derivatives have previously been proposed for use as herbicides and fungicides, and for example, the compounds disclosed in European patent applications, publication numbers 59536, 61836 and 76030, may be mentioned.

We have now found that a certain, related, class of compounds possess an appreciably longer persistence of effect against certain fungicidal diseases of plants.

According to the present invention therefore there are provided amide derivatives of the general formula (I):

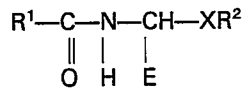

FORMULA I

wherein

$R^1$ is an optionally substituted aryl or heterocyclyl radical;

X is oxygen or sulphur; and

E is CN, $CONH_2$ or $CSNH_2$; characterised in that $R^2$ is a $C_3$ to $C_7$ alkynyl group optionally substituted by $C_{1-4}$ alkyl, chlorine, bromine or iodine.

When $R^1$ is an optionally substituted aryl group it may be a phenyl or naphthyl radical. Examples of substituents which may be present include $C_1$—$C_4$ alkyl, fluorine, chlorine, bromine, iodine, $C_1$—$C_4$ alkoxy, methylenedioxy and ethylenedioxy, $C_1$—$C_4$ alkylthio, $C_1$—$C_4$ haloalkyl (eg. $CF_3$) nitro and cyano. There may be from one to three or more substituents which may be the same or different. When $R^1$ is a substituted phenyl radical the substituents are preferably in the 3, 4 or 5 positions. When a methylenedioxy or ethylenedioxy substituent is present, it preferably is attached to the 3 and 4 positions of the phenyl ring. A halogen substituent (eg. Cl or Br) may also be present in the 4- or 5-position, or both, in such compounds.

When $R^1$ is an optionally substituted heterocyclyl radical, it may for example be a furyl, benzfuryl, thienyl, pyridyl, thiazolyl, or benzthiazolyl radical. Examples of substituents which may be present include those listed above for the case when $R^1$ is a substituted phenyl radical. There may be from one to three or more substituents which may be the same or different.

The structural formula (I) given above is believed to be the one which best represent the structure of the compounds. For some compounds within the scope of the formula (I) it may be possible for tautomeric forms of the compound to exist, in which a hydrogen atom is transposed to another part of the molecule and the chemical bonds between the atoms of the molecule are consequently rearranged. Thus it is possible for the molecule to exist in the alternative form (II):

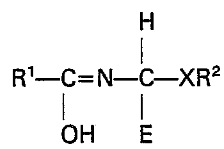

FORMULA II

The structural formula (I) is intended to represent and include such tautomeric forms, insofar as they may exist. The structural formula (I) is also intended to include any physically distinguishable modifications of the compounds which may arise, for example, from different ways in which the molecules are arranged in a crystal lattice, or from the inability of parts of the molecules to rotate freely in relation to other parts, or from geometrical and or optical isomerism, or from intramolecular or intermolecular bonding, or otherwise.

Particular examples of compounds according to the invention are listed in Table I below:

TABLE I

| Compound No. | $R^1$ | $XR^2$ | E | Route | Melting Point °C |
|---|---|---|---|---|---|
| 1 | 4-Cl.$C_6H_4$ | $OCH_2C{\equiv}CH$ | $CONH_2$ | A | 124—126 |
| 2 | 4-Cl.$C_6H_4$ | $OCH_2C{\equiv}CH$ | CN | A | 84—87 |
| 3 | 4-Br.$C_6H_4$ | $OCH_2C{\equiv}CH$ | $CONH_2$ | A | 130—132 |
| 4 | 4-Br.$C_6H_4$ | $OCH_2C{\equiv}CH$ | CN | A | 96—98 |
| 5 | 4-$CH_3O$.$C_6H_4$ | $OCH_2C{\equiv}CH$ | $CONH_2$ | A | |
| 6 | 4-$CH_3O$.$C_6H_4$ | $OCH_2C{\equiv}CH$ | CN | A | |
| 7 | 4-$CH_3$.$C_6H_4$ | $OCH_2C{\equiv}CH$ | $CONH_2$ | A | |
| 8 | 4-$CH_3$.$C_6H_4$ | $OCH_2C{\equiv}CH$ | CN | A | |
| 9 | 4-CN.$C_6H_4$ | $OCH_2{\equiv}CH$ | $CONH_2$ | A | |
| 10 | 4-CN.$C_6H_4$ | $OCH_2C{\equiv}CH$ | CN | A | |
| 11 | 4-Cl.$C_6H_4$ | $OCH_2C{\equiv}CCH_3$ | $CONH_2$ | A | 131—134 |
| 12 | 4-Cl.$C_6H_4$ | $OCH_2C{\equiv}CCH_3$ | CN | A | 92—93 |
| 13 | 4-Cl.$C_6H_4$ | $OC(CH_3)_2C{\equiv}CH$ | $CONH_2$ | A | |
| 14 | 4-Cl.$C_6H_4$ | $OC(CH_3)_2C{\equiv}CH$ | CN | A | 143—145 |
| 15 | 4-Cl.$C_6H_4$ | $OCH_2C{\equiv}CI$ | $CONH_2$ | A | |
| 16 | 4-Cl.$C_6H_4$ | $OCH_2C{\equiv}CI$ | CN | A | |
| 17 | 4-Cl.$C_6H_4$ | $OCH_2C{\equiv}CBr$ | $CONH_2$ | A | |
| 18 | 4-Cl.$C_6H_4$ | $OCH_2C{\equiv}CBr$ | CN | A | |
| 19 | 4-Br.$C_6H_4$ | $OCH_2C{\equiv}CH$ | $CSNH_2$ | E | 145—146 |
| 20 | 4-Cl.$C_6H_4$ | $OCH_2C{\equiv}CH$ | $CSNH_2$ | E | |
| 21 | 4-$CH_3$.$C_6H_4$ | $OCH_2C{\equiv}CH$ | $CSNH_2$ | E | |
| 22 | 4-Cl.$C_6H_4$ | $OCH(CH_3)C{\equiv}CH$ | CN | D | |
| 23 | 4-$CF_3$.$C_6H_4$ | $OCH_2C{\equiv}CH$ | CN | A | |
| 24 | 4-$NO_2$.$C_6H_4$ | $OCH_2C{\equiv}CH$ | CN | A | |
| 25 | 4-$C_2H_5$.$C_6H_4$ | $OCH_2C{\equiv}CH$ | CN | A | |
| 26 | 4-$C_2H_5O$.$C_6H_4$ | $OCH_2C{\equiv}CH$ | CN | A | |
| 27 | 2-Cl-5-pyridyl | $OCH_2C{\equiv}CH$ | CN | D | 110—112 |
| 28 | 2-naphthyl | $OCH_2C{\equiv}CH$ | CN | A | |
| 29 | 2-Cl-5-thienyl | $OCH_2C{\equiv}CH$ | CN | A | |
| 30 | 2-Br-5-furyl | $OCH_2C{\equiv}CH$ | CN | A | |
| 31 | 4-Cl.$C_6H_4$ | $SCH_2C{\equiv}CH$ | Cn | D | |

The invention further provides processes for preparing compounds of formula (I) above. Thus the compounds may be prepared, for example, by the process of Scheme A below:

Scheme A

(a) $R^1COCl + NH_2CH_2CN \rightarrow R^1CONHCH_2CN$ (III)

(b) 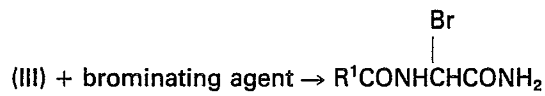

(IV)

(c) 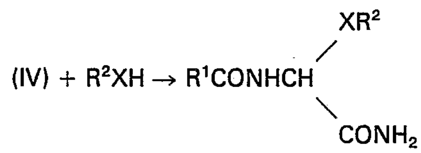
(V)

(d) 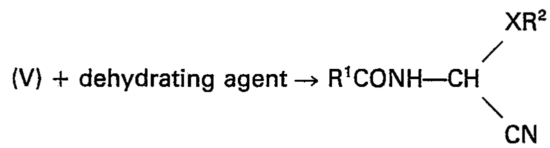

(VI)

The process outlined in Scheme A begins with step (a), in which an acid chloride $R^1COCl$ is reacted with aminoacetonitrile by a conventional procedure to obtain the acylaminoacetonitrile derivative (III). This is then reacted in step (b) with a brominating agent (for example bromine in glacial acetic acid) to give the brominated derivative (IV). This bromination procedure also simultaneously hydrates the cyano group to a carbamoyl group $—CONH_2$, and necessitates treatment with a dehydrating agent at a later stage to convert the carbamoyl group back into a cyano group. It may be possible to avoid the undesired conversion of the cyano group to carbamoyl by use of a different solvent or brominating agent and thereby shorten the process by making step (d) unnecessary.

In step (c), the bromo compound (IV) is reacted with an appropriate alcohol or thiol, or formula $R^2XH$ to obtain the carbamoyl compound (V). Preferably the reaction is carried out in a solvent; the solvent should be an aprotic solvent to avoid reaction of the solvent with the bromo-compound (IV). Preferably an acid acceptor is present in at least a stoichiometric proportion. Examples of acid acceptors include tertiary amines, for example triethylamine and pyridine. The reaction takes place readily even at ambient temperatures but may be accelerated if desired by heating for example to 100°C or above.

The final step (d) of Scheme A is the treatment of the carbamoyl compound (V) with a dehydrating agent to convert it to the corresponding cyano compound. The dehydrating agent may be, for example, a bi-molar amount of *p*-toluene sulphonyl chloride in pyridine as solvent and acid acceptor, or another dehydrating agent, for example phosphorus oxychloride-dimethylformamide. The reaction with *p*-toluenesulphonyl chloride proceeds readily at ambient temperature. Scheme A has been described in terms of brominated compounds; however, the scheme could equally be carried out using a chlorinating agent (eg. gaseous chlorine) in place of a brominating agent, to produce the chlorinated compound corresponding to compound (IV); this could then be used in step (c) in place of compound (IV). This route cannot be used where $R^1$ is readily attacked by elemental bromine or chlorine.

A further process for making compounds of the invention is outlined in Scheme B, wherein $R^6$ is $C_1—C_6$ alkyl:

Scheme B

(a) $R^1CONH_2 + HCO—CO_2R^6 \rightarrow R^1CONH—CH(OH)CO_2R^6$ (VII)

(b) $(VII) + SOCl_2 \rightarrow R^1CONHCH(Cl)CO_2R^6$ (VIII)

(c) 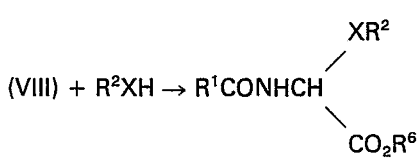
(IX)

(d) $(IX) + NH_3 \rightarrow (V)$

In step (a) of Scheme B, an amide $R^1CONH_2$ is condensed with a glyoxylic ester $HCO—CO_2R^6$ to give the hydroxy intermediate (VII). The group $R^6$ is an ester radical, for example an alkyl group of 1 to 4 carbon atoms (eg. a methyl group). In step (b), the hydroxy intermediate (VII) is treated with a chlorinating agent (eg. thionyl chloride) to convert it to the chloro-derivative (VIII). This is in turn reacted in step (c) with the appropriate alcohol, or thiol, $R^2XH$ to give the ester (IX). Treatment of this with ammonia in step (c) gives the carbamoyl derivative (V) which may then be converted to the cyano compound of the invention by the method of step (d) of Scheme A.

An alternative process for preparing the amide derivatives of the invention when X=0 in formula I is a variation of the Scheme B process above. This Scheme B variation is outlined below:

(a)

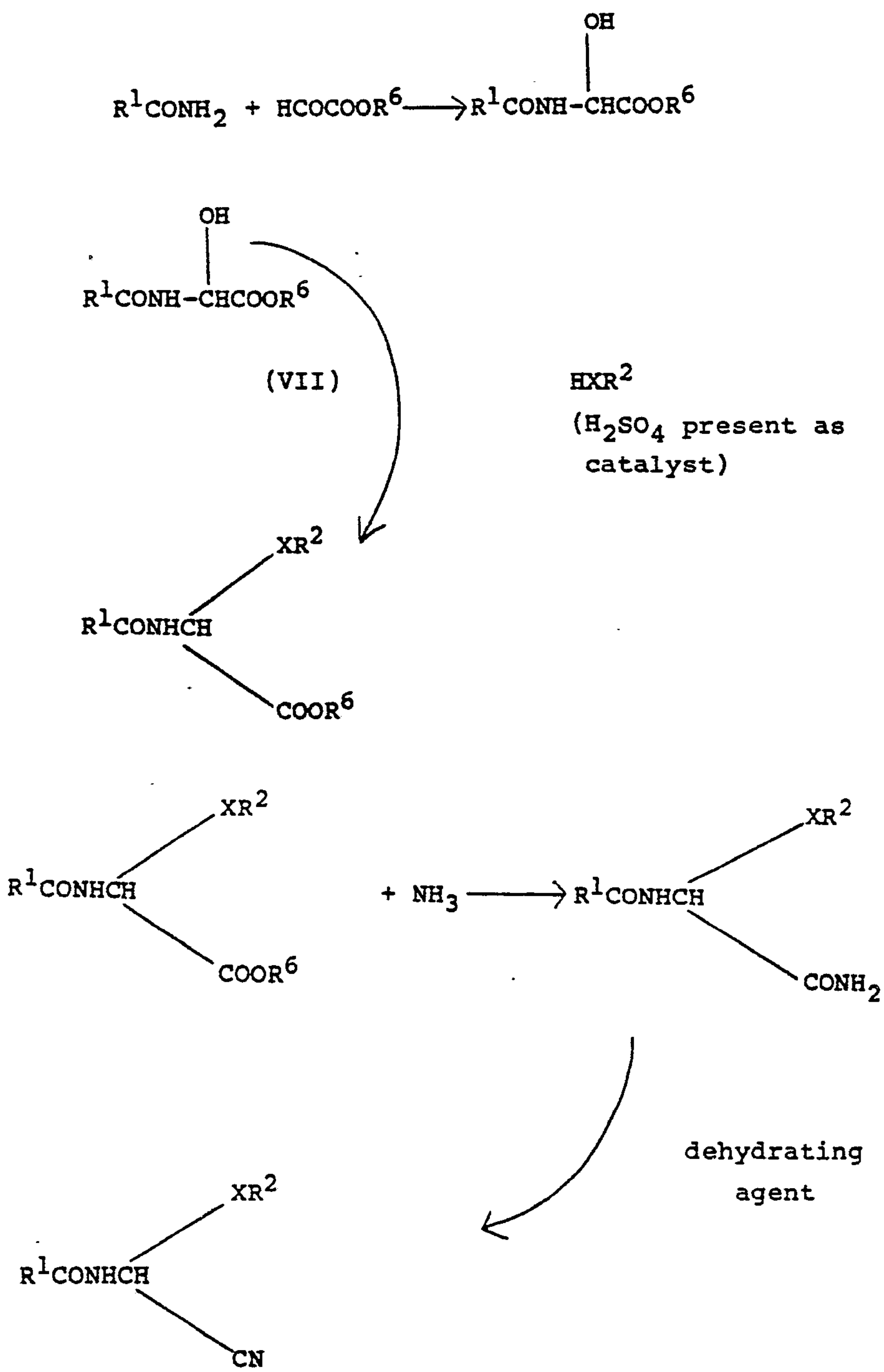


(VII)

(b)

(VII)

(IX)

(c)

(V)

(VI)

In this modified Scheme B procedure it can be seen that one can proceed directly from the hydroxy intermediate (VII) to the ester (IX) in one step. The ester (IX) is then treated with ammonia, as in step c, to produce the carbamoyl derivative (V) which in turn is converted to the cyano compound of the invention (VI) by means of the final step (step d) of the process of Scheme A.

Scheme C

(a)

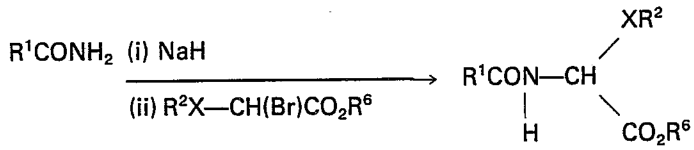


(X)

(b)

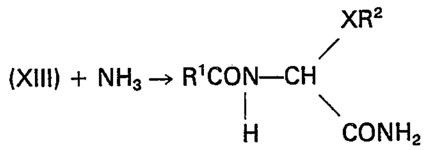


(XI)

(c)

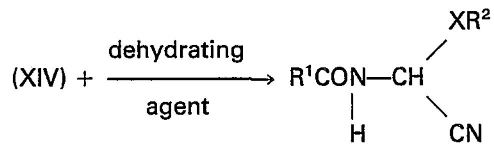


(XII)

According to Scheme C, an amide $R^1CONH_2$ is first treated with sodium hydride and the anion so generated is then reacted with an alpha bromo ester $R^2X—CH(Br)CO_2R^6$ to give the ester (X). This is then reacted with ammonia to give the amide (XI), and finally (XI) is treated with a dehydrating agent to give the nitrile (XII).

A further method for preparing compounds of the invention is outlined in Scheme D.

Scheme D

(a)

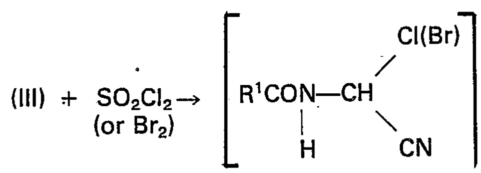


(XIII)

(b)

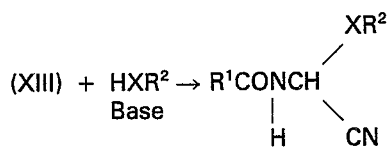


(XII)

According to Scheme D (III) is chlorinated (eg. with $SO_2Cl_2$) or brominated (eg. with $Br_2$) in an aprotic solvent to give the highly reactive bromo- or chloro-derivative (XIII). This is treated with the appropriate alcohol or thiol in the presence of base to give the required nitrile (XII).

The chloro- or bromo- nitriles (XIII) are too unstable to be isolated and characterised, and are used within a short time after they are prepared. The final stage (b) of the scheme may conveniently be carried out by using an excess of the alcohol or thiol, and anhydrous potassium carbonate as the base. Triethylamine or other tertiary amines may also be used as the base.

Compounds of the invention in which the group E is a thiocarbamoyl radical may be prepared according to Scheme E below:

Scheme E

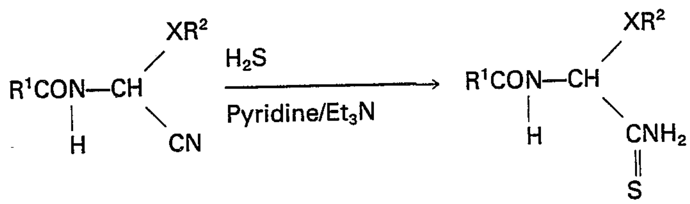


(XIV)

The reaction is conveniently carried out by passing gaseous $H_2S$ through a solution of the nitrile in a suitable solvent such as toluene or pyridine containing a little triethylamine as catalyst. Usually the solution

is externally cooled to 0—10°. If the product does not separate from the solution, it may be isolated by removal of the solvent.

The amide derivatives of formula I, and compositions containing them, are variously active against a wide range of fungal diseases, particularly, for example, against:

*Plasmopara viticola* (downy mildew) on vines and
*Phytophthora infestans* (late blight) on potatoes and tomatoes and other species of *Phytophthora*
*Phytophthora parasitica, Phytophthora cinnamomi, Phytophthora palmivora* and *Phytophthora capsici* on a range of commercially important crops
*Pseudoperonospora cubensis* on cucurbits
*Peronospora tabacina* on tobacco
*Peronospora parasitica* on cabbage
*Peronospora destructor* on onions
*Bremia lactuca* on lettuce
*Pythium* species on a range of commercially important crops

Other downy mildews and other fungal diseases, for example:

*Venturia inaequalis* (scab) on apples
*Cercospora arachidicola* on peanuts and other *Cercospora* species, *Pyricularia* on rice, *Xanthiomonas* on rice and *Fusaria* cudmaria.

A particularly valuable feature of the activity of the amide derivatives is their systemic effect, ie. their ability to move in a plant to combat an infection or infestation remote from the site of initial application. Thus a derivative, or a composition containing it, may be applied to the soil surrounding the roots of a plant or to the seed or to other plant areas, eg. leaves, and be taken up by the plant through its roots, or other areas, to combat fungi locally or elsewhere on the plants.

In another aspect, therefore, the invention provides a process for combatting fungi, especially of inhibiting the growth of fungi on plants or seeds, which comprises applying to the plants, or the locus thereof, or to seeds, a fungicidally effective amount of a compound of the formula (I) as hereinbefore defined. The amount of the compound may vary, depending upon the identity of the particular compound chosen, the fungal species whose growth is to be inhibited, and the plant or locus involved.

The skilled worker in the fungicide art will readily be able to establish appropriate application rates by standard procedures without undue experimentation.

Preferred compounds for use in the fungicidal compositions of the invention and the process for combatting fungi are those defined in detail above with reference to formula I wherein $R^1$ is optionally-substituted phenyl, or heterocyclyl, for example 2-chloro-5-pyridyl, X is O or S (X is preferably O) and $R^2$ is optionally substituted $C_3$—$C_7$ alkynyl, and E is CN or $CSNH_2$.

Preferred phenyl substitution, when $R^1$ is phenyl, is at the 3, 4 or 5 positions and is alkyl, alkoxy, methylenedioxy or halogen.

The compounds used in the process and compositions of the invention are preferably applied in the form of a composition, in which the active ingredient is mixed with a carrier comprising a solid or liquid diluent. In another aspect, therefore, the invention provides a fungicidal composition, comprising as an active ingredient a compound of the formula (I) as hereinbefore defined, in admixture with a solid or liquid diluent. Preferably the composition also comprises a surface-active agent.

The amide derivatives may be used as such for anti-fungal purposes but are more conveniently formulated into compositions for such usage.

The amide derivatives and compositions containing them can be used to combat plant fungi and treat plants or seeds in a number of ways, for example they can be applied, formulated or unformulated, directly to the foliage of a plant which is infected or likely to become infected, or they can be applied also to bushes and trees, to soil or to other medium in which plants, bushes or trees are growing or to be planted, or they can be sprayed on, dusted on or applied as a cream or paste formulation. Application can be to any part of the plant, bush or tree, for example to the foliage, stems, branches, seeds or roots, or to the soil surrounding the roots.

The terms "combatting" and "treatment" as used herein embrace all the foregoing modes of application and the term "plant" includes seedlings, bushes and trees. Furthermore, the method of the invention includes protectant, prophylactic and eradicant treatment.

The derivatives are preferably used for agricultural and horticultural purposes in the form of compositions. The type of composition used in any instance will depend upon the particular purpose envisaged.

The compositions may be in the form of dusting powders or granules, for example ordinary grains or "slow release" granules wherein the active ingredient is mixed with a solid diluent or carrier, for example, a natural clay including kaolin, bentonite, kieselguhr, dolomite, Fuller's earth, gypsum, Hewitt's earth, diatomaceous earth and China clay; or powdered magnesia, silica or talc.

Compositions for dressing seed may, for example, comprise an agent (for example a mineral oil) for assisting the adhesion of the composition to the seed.

The compositions may also be in the form of dispersible powders or grains comprising a wetting agent to facilitate the dispersion in liquids of the powder or grains which may contain also fillers and suspending agents.

The aqueous dispersion of emulsions may be prepared by dissolving the active ingredient(s) in an organic solvent which may contain wetting, dispersing or emulsifying agent(s) and then adding the mixture so obtained to water which may also contain wetting, dispersing or emulsifying agent(s). Suitable organic solvents are ethylene dichloride, isopropyl alcohol, propylene glycol, diacetone alcohol, toluene, kerosene, methylnaphthalene, xylenes and trichloroethylene.

The compositions for spraying may also be in the form of aerosols wherein the formulation is held in a propellant, eg. fluorotrichloromethane or dichlorodifluoromethane.

By including suitable additives, for example additives for improving the distribution, adhesive power and resistance to rain on treated surfaces, the different compositions can be better adapted for various utilities.

The derivatives can be used in smoke generators and also as mixtures with fertilisers (eg. nitrogen- or phosphorus- containing fertilisers). Compositions comprising only granules of fertiliser incorporating, for example coated with, the derivative, are preferred.

The invention therefore also provides a fertiliser composition comprising the derivative and a fertiliser.

The compositions may also be in the form of liquid preparations for use as dips or sprays which are generally aqueous dispersions or emulsions containing the active ingredient in the presence of one or more surface active agent(s), dispersing agent(s), emulsifying agent(s) or anionic or non-ionic agents. Suitable cationic agents are quaternary ammonium compounds for example, cetyltrimethylammonium bromide.

Suitable anionic agents are soaps, salts of aliphatic monoesters of sulphuric acid (for example sodium lauryl sulphate), and salts of sulphonated aromatic compounds (for example sodium dodecylbenzenesulphonate, sodium, calcium or ammonium lignosulphonate, butylnaphthalene sulphonate, and a mixture of sodium di-isopropyl- and triisopropylnaphthalene sulphonates).

Suitable non-ionic agents are the condensation products of ethylene oxide with fatty alcohols such as oleyl alcohol or cetyl alcohol, or with alkyl phenols such as octylphenol, nonylphenol and octylcresol. Other non-ionic agents are the partial esters derived from long chain fatty acids and hexitol anhydrides, the condensation products of the said partial esters with ethylene oxide, and the lecithins. Suitable suspending agents are hydrophilic colloids (for example polyvinylpyrrolidone and sodium carboxymethylcellulose), and the vegetable gums (for example gum acacia and gum tragacanth).

The compositions for use as aqueous dispersions or emulsions are generally supplied in the form of a concentrate containing a high proportion of the active ingredient(s), the concentrate to be diluted with water before use. These concentrates often should be able to withstand storage for prolonged periods and after such form aqueous preparations which remain homogenous for a sufficient time to enable them to be applied by conventional spray equipment. The concentrates may conveniently contain 10—85%, generally 25—60%, by weight of the active ingredient(s).

When diluted to form aqueous preparations, such preparations may contain varying amounts of the active ingredient(s) depending upon the intended purpose, but an aqueous preparation containing 0.0005% or 0.01% to 10% by weight of active ingredient(s) may be used.

The compositions of this invention can comprise also other compound(s) having biological activity, eg. compounds having similar or complementary fungicidal or plant growth regulating activity or compounds having herbicidal or insecticidal activity.

The other fungicidal compound can be for example one which is capable of combating ear diseases of cereals (eg. wheat) such as *Septoria, Gibberella* and *Helminthosporium* spp., seed and soil borne diseases and downy and powdery mildews on grapes and powdery mildew and scab on apple etc or cercospora on a variety of crops, or other diseases such as *Cercosporella herpotrichoides* on cereals. These mixtures of fungicides can have a broader spectrum of activity than the compound of general formula (I) alone; further the other fungicide can have a synergistic effect of the fungicidal activity of the compound of general formula (I). Examples of the other fungicidal compound are imazalil, benomyl, carbendazim (BCM), thiophanate-methyl, captafol, captan, folpet, sulphur, dithiocarbamates, carbathiins, dicarboximides (including iprodione, vinchlozolin, procymidone), copper oxychloride, triforine, dodemorph, tridemorph, dithianon, pyrazophos, binapacryl, "Topaz", fenarimol, quinomethionate, panoctine, furalaxyl, aluminium tris(ethylphosphonate), cymoxanil, ethirimol, dimethirimol, bupirimate, metalaxyl, oxadixyl, benaboxyl, ofurace, chlorothalonil, metazanine and ergosterol-synthesis inhibiting fungicides other than those here disclosed.

Suitable insecticides are pirimor, croneton, dimethoate, metasystox, formothion, and pyrethroid compounds.

The other plant growth regulating compound can be one which controls weeds or seedhead formation, improves the level or longevity of the plant growth regulating activity of the compounds of general formula (I), selectively controls the growth of the less desirable plants (eg. grasses) or causes the compound of general formula (I) to act faster or slower as a plant growth regulating agent. Some of these other agents will be herbicides. Examples of suitable agents are the gibberellins (eg. $GA_3$, $GA_4$ or $GA_7$), the auxins (eg. indoleacetic acid, indolebutyric acid, naphthoxyacetic acid or naphthylacetic acid); the cytokinins (eg. kinetin, diphenylurea, benzimidazole, benzyladenine or BAP), phenoxyacetic acids (eg. 2,4-D or MCPA), substituted benzoic acids (eg. TIBA), morphactins (eg. chlorfluorecol), maleic hydrazide, glyphosate, glyphosine, long chain fatty alcohols and acids (eg. Off Shoot O or Off Shoot T), dikegulac, Sustar, Embark,

substituted quaternary ammonium and phosphonium compounds (eg. CCC or Phosfon-D), Ethrel, carbetamide, Racuza, Alar, asulam, abscissic acid, isopyrimol, RH531, hydroxybenzonitriles (eg. bromoxynil), Avenge, Suffix or Lontrel.

The invention is illustrated by the following Examples, in which unless otherwise stated all parts are by weight and temperatures in degrees Celsius. The Examples that describe chemical syntheses give details in some cases of the nuclear magnetic resonance (NMR) spectra of the compounds. The information given is the chemical shift (δ) for each peak in the spectrum together with a symbol to indicate the nature of the peak, as follows: s(singlet); d(doublet); m(multiplet); q(quartet); t(triplet). The solvent used was fully deuterated dimethyl sulphoxide of deuterochlorform ($CDCl_3$). Information is also given on infra-red (IR) spectra of the compounds. The information given is the transmission for each peak together with a symbol to indicate the size of the peak; s(strong); w(weak).

Example 1

This Example illustrates the preparation of compound no. 1 of Table I by the process of Scheme A.

(a) Preparation of 4-chlorobenzoylamino(bromo)acetamide

To a suspension of 4-chloro benzoylaminoacetonitrile (5g) in stirred glacial acetic acid (50ml) was added all at once bromine (5.0g). After a brief induction period, the bromine colour was discharged and the product (5.8g) precipitated from the acetic acid. The product was filtered off, washed with glacial acetic acid, and then with anhydrous ether, and dried. A sample crystallised from glacial acetic acid had m.p. 157° (dec).

(b) Preparation of 4-chlorobenzoylamino (propargyloxy) acetamide

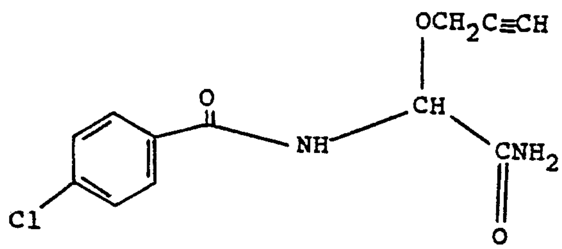

The bromoamide (10.5g) prepared according to (a) above was added in one portion to propargyl alcohol stirred at room temperature for 3 hours and then stood for 48 hours. Excess alcohol was evaporated under reduced pressure and the brown oil obtained triturated with ethyl acetate/ether to give the product as a brown solid (2.4g) mpt 123—5°C. The filtrate deposited more solid which was washed with chloroform to give a white powder, (0.9g) mpt 124—6°C.

NMR(DMSO-d6) δ 3.40 (t, 1H), 4.21 (d, 2H), 5.60 (d, 1H), 7.45 (S, 2H), 7.50 (d, 2H), 7.90 (d, 2H), 9.20 (d, 1H).

IR (nujol) $cm^{-1}$ 3470 (s), 3250 (b), 2125 (w), 1680 (b)

Example 2

This Example illustrates the preparation of

4-chlorobenzoylamino(propargyloxy)acetonitrile having the structural formula:

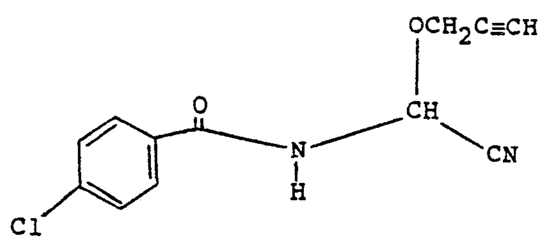

Phosphorus oxychloride (1.07g) was added dropwise to dry DMF(5ml) at 0°C. After 15 minutes this solution was added in one portion to a stirred solution of the propargyl amide (1.50 g) prepared according to Example 1 in dry DMF (15 ml) at 0°C. The resulting brown solution was stirred at 0°C for 20 minutes, then poured into water (200 ml) and extracted twice with ether (50 ml). The ether extracts were washed with water, dried over magnesium sulphate and evaporated to give a brown oil, (1.1 g). This was purified by flash chromatography on silica gel, eluting with ethyl acetate, to give the product as pale orange crystals (0.78 g), mpt 84—7°C.

NMR ($CDCl_3$) δ 2.56 (t, 1H), 4.43 (d, 2H), 6.50 (d, 1H), 7.48 (d, 2H), 7.90 (d, 2H) 8.30 (d, 1H)

IR (nujol $cm^{-1}$ 3290 (sd), 2120 (w), 1670 (s)

Example 3

This Example illustrates the preparation of compound no 3 of table I, having the chemical name

Preparation of 4-bromobenzoylamino(propargyloxy)acetamide and having the structural formula:

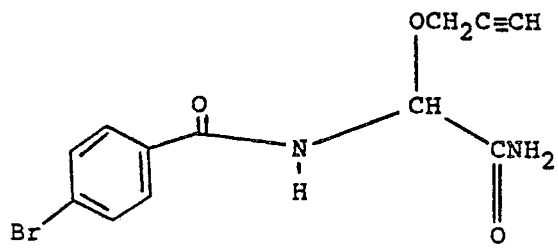

The corresponding bromoamide (10.0g) prepared similarly to the method of Example I was suspended in propargyl alcohol and the mixture stirred for 6 hours and then stood overnight. A small amount of solid was filtered, and the filtrate evaporated under reduced pressure. The residual oil and solid was stirred for 1 hour with 10% ammonium hydroxide (50ml). The solid suspension was then filtered and dried over $P_2O_5$ to give a buff coloured solid (6.07g). Recrystallisation from toluene gave a buff solid (4.72g) mpt 130—132°C.

NMR (DMSO-d6) δ 3.40 (t, 1H), 4.20 (d, 2H), 5.56 (d, 1H), 7.40 (bs, 2H), 7.62 (d, 2H), 7.80 (d, 2H), 9.18 (d, 1H)

IR (nujol) $cm^{-1}$ 3470 (s), 3270 (s), 3230 (s), 2150 (w), 1715 (s), 1650 (s)

Example 4

This Example illustrates the preparation of compound no 4 of table I having the chemical name Preparation of 4-bromobenzoylamino(propargyloxy)-acetonitrile and having the structural formula:

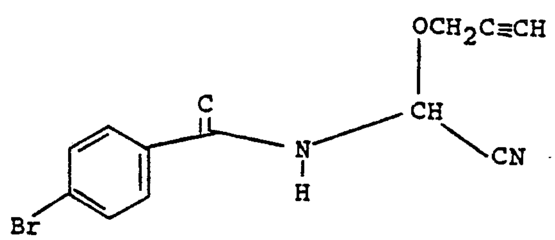

Phosphorus oxychloride (1.95g) was added to dry DMF (4ml) at 0°C, and the solution stirred for 10 minutes, and then added to the propargyl amide (3.11g) prepared according to Example 3 in dry DMF (4ml) at 0°C. After the initial exothermic reaction the mixture was cooled to 0°C and stirred for 1 hour. It was then poured into water and the mixture extracted with ether, and the ethereal extracts dried over magnesium sulphate and evaporated to yield a brown oil which crystallised (2.96g). The solid was purified by flash chromatography on silica gel, eluting with light petroleum (60:80)/ethyl acetate 6:1, to give white crystals (2.05g), mpt 96—98°C.

NMR ($CDCl_3$) δ 2.58 (t, 1H), 4.39 (d, 2H), 6.44 (d, 1H), 7.65 (m, 4H)

IR (nujol) $cm^{-1}$ 3300 (s), 2110 (w), 1675 (s)

Example 5

Preparation of 2-chloro-5-pyridinecarbonylamino(proparglоxy)acetonitrile and having the structural formula:

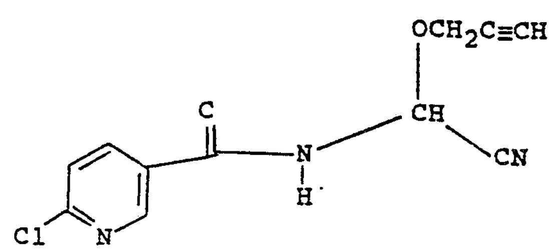

To a stirred solution of 2-chloro-5-pyridinecarbonylamino acetonitrile (1.96g) in dry ethyl acetate (25 ml) at 42°C, was added bromine (1.6g) dropwise. After completion of the addition the mixture was stirred for 10 minutes and then filtered. To the filtrate was added propargyl alcohol (5ml) followed by triethylamine (2.02g) in dry ethyl acetate (35 ml). The white precipitate at once formed was filtered, the filtrate washed with water, dried over $MgSO_4$ and evaporated to give a red-brown oil.

The compound was purified by HPLC on Merck 9385 silica gel, eluting with light petroleum (60:80)/ethyl acetate 3:1, to give an oil which crystallised. Recrystallisation from ether/petrol afforded a white solid (0.21g) mpt = 110—112°C.

NMR ($CDCl_3$) δ 2.60 (t, 1H); 4.39 (d, 2H); 6.39 (d, 1H); 7.40—7.60 (m, 1H); 8.39 (d, 1H); 8.84 (m, 1H).
IR (nujol) 3300 (sharp), 3260 (S), 2120 (W), 1670 (S).

Example 6

This Example illustrates a composition according to the invention which comprises an emulsifiable concentrate. The following ingredients were thoroughly mixed to give a solution.

| | |
|---|---|
| Compound No. 1 of Table I | 10% |
| Ethylene dichloride | 40% |
| Calcium dodecyclbenzenesulphate | 5% |
| "Lubrol" L | 10% |
| "Aromasol" H | 35% |

Example 7

A composition in the form of grains readily dispersible in a liquid, eg. water, was prepared by grinding together the first three ingredients in the presence of added water and then mixing in the sodium acetate. The resultant mixture was dried and passed through a British Standard mesh sieve, size 44—100, to obtain the desired size of grains.

| | |
|---|---|
| Compound No. 2 of Table I | 50% |
| "Dispersol" T | 25% |
| "Lubrol" APN 5 | 1.5% |
| Sodium acetate | 23.5% |

Example 8

The following ingredients were ground together to produce a powder formulation readily dispersible in liquids.

| | |
|---|---|
| Compound No. 3 of Table I | 45% |
| "Dispersol" T | 5% |
| "Lissapol" NX | 0.5% |
| "Cellofas" B600 | 2% |
| Sodium acetate | 47.5% |

Example 9

The active ingredient was dissolved in acetone and the resultant liquid was sprayed on to the granules of china clay. The solvent was then allowed to evaporate to produce a granular composition.

| | |
|---|---|
| Compound No. 4 of Table I | 5% |
| China clay granules | 95% |

Example 10

A composition suitable for use as a seed dressing was prepared by mixing the following three ingredients.

| | |
|---|---|
| Compound No. 1 of Table I | 50% |
| Mineral oil | 2% |
| China clay | 48% |

Example 11

A dusting powder was prepared by mixing the active ingredient with talc.

| | |
|---|---|
| Compound No. 2 of Table I | 5% |
| Talc | 95% |

Example 12

A Col formulation was prepared by ball-milling the constituents set out below and then forming an aqueous suspension of the ground mixture with water.

| | |
|---|---|
| Compound No. 3 of Table I | 40% |
| "Dispersol" T | 10% |
| "Lubrol" APN5 | 1% |
| Water | 49% |

Example 13

A dispersible powder formulation was made by mixing together the ingredients set out below and then grinding the mixture until all were thoroughly mixed.

| | |
|---|---|
| Compound No. 4 of Table I | 25% |
| "Aerosol" OT/B | 2% |
| "Dispersol" A.C. | 5% |
| China clay | 28% |
| Silica | 40% |

Example 14

This Example illustrates the preparation of a dispersible powder formulation. The ingredients were mixed and the mixture then ground in a comminution mill.

| | |
|---|---|
| Compound No. 1 of Table I | 25% |
| "PERMINAL" BX | 1% |
| "Dispersol" T | 5% |
| Polyvinylpyrrolidone | 10% |
| Silica | 25% |
| China clay | 34% |

Example 15

The ingredients set out below were formulated into a dispersible powder by mixing then grinding the ingredients.

| | |
|---|---|
| Compound No. 2 of Table I | 25% |
| "Aerosol" OT/B | 2% |
| "Dispersol" A | 5% |
| China clay | 68% |

In Examples 5 to 14 the proportions of the ingredients given are by weight and the Examples were all repeated using, as active ingredient, the other compounds of Table I.

There now follows an explanation of the compositions or substances represented by the various Trade Marks and Trade Names mentioned above.

| | |
|---|---|
| LUBROL L : | a condensate of nonyl phenol (1 mole) with ethylene oxide (13 moles). |
| AROMASOL H : | a solvent mixture of alkyl-benzenes |
| DISPERSOL T AND AC : | a mixture of sodium sulphate and a condensate of formaldehyde with sodium naphthalene sulphonate |
| LUBROL APN 5 : | a condensate of nonyl phenol (1 mole) with ethylene oxide (5.5 moles) |
| CELLOFAS B600: | a sodium carboxymethyl cellulose thickener. |

Example 16

The compounds were tested against a variety of mainly foliar fungal diseases of plants. The techniques were as follows:

For all tests, plants were grown in John Innes Potting Compost (No 1 or 2) in 4cm diameter minipots. The test compounds were formulated either by bead milling with aqueous Dispersol T or as a solution in acetone or acetone/ethanol which was diluted to the required concentration immediately before use. For the foliar diseases, solutions and suspensions (100ppm ai) were sprayed on the foliage and applied to the roots of the plant via the soil. The sprays were applied to maximum retention and the root drenches to a

final concentration equivalent to approximately 40ppm ai/dry soil. Tween 20, to give a final concentration of 0.05%, was added when the sprays were applied to cereals (ai means "active ingredient").

Most were proctectant tests where the compound was applied to the soil and roots and to the foliage one or two days before the plant was inoculated with the pathogen. However, in the case of the test against *Erysiphe graminis hordei* the treatment was eradicative and the compounds were applied one day after inoculation.

The foliar pathogens were applied by spraying spore suspensions onto the leaves of the test plants. Cultures of the soil pathogens were mixed with vermiculite into which seed was sown prior to drenching with chemical.

After inoculation, the plants or pots with seed were placed in an appropriate environment to allow infection to proceed and then incubated until the disease was ready for assessment. The period between inoculation and assessment varied from four to fourteen days according to the disease and the environment.

Disease control was recorded using the following grading system:

4 = no disease
3 = trace to 5% of disease on untreated plants
2 = 6—25% of disease on untreated plants
1 = 26—59% of disease on untreated plants
0 = 60—100% of disease on untreated plants

TABLE II

| Compound Number | Vi | Po | Ca | Pv | Xo | Fc | Pu |
|---|---|---|---|---|---|---|---|
| 1 | 0 | 0 | 0 | 0 | 0 | 0 | 0 |
| 2 | 4 | 0 | 3 | 4 | 0 | 0 | 3 |
| 3 | 0 | 0 | 0 | 0 | 0* | 0 | 0 |
| 4 | 0 | 0 | 2 | 4 | 0* | 2 | 1 |
| 11 | 0 | 0 | 0 | 0 | 3 | 2 | 0 |
| 12 | 0 | 2 | 0 | 0 | 0 | 0 | 0 |
| 14 | 0 | 0 | 0 | 3 | 0 | — | — |
| 19 | 0 | 0 | 3 | 3 | 0* | 0 | 4 |
| 22 | 0 | 2 | 0 | 3 | 0 | — | — |

A dash, thus "—", in the table in any column indicates that the particular compound was not tested against that particular disease.
* tested against Xm
Vi = *Venturia* inaequalis
Po = *Pyricularia* oryzae
Ca = *Cercospora* arachidicola
Pv = *Plasmopara* viticola
Xo = *Xanthomonas* oryzae
Fc = *Fusarium* culmoru
Pu = *Pythium* ultima
Xm = *Xanthomonas* malvacearum

**Claims for the Contracting States: BE CH DE FR GB IT LI NL**

1. A compound of the general formula:

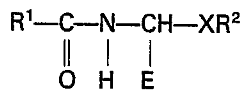

or a tautomer thereof, wherein:

$R^1$ is an aryl or substituted aryl radical, or a heterocyclyl or substituted heterocycyclyl radical;

X is oxygen or sulphur, and

E is CN, $CONH_2$ or $CSNH_2$; characterised in that $R^2$ is a $C_3$ to $C_7$ alkynyl group optionally-substituted by $C_{1-4}$ alkyl, chlorine, bromine or iodine.

2. A compound according to claim 1, wherein $R^1$ is phenyl or substituted phenyl.

3. A compound according to claim 1, wherein $R^1$ is naphthyl or substituted naphthyl.

4. A compound according to claim 1, wherein $R^1$ is is an aryl or heterocyclyl radical substituted with one or more groups, which substituted group or groups may be chlorine, bromine or fluorine; $C_{1-4}$ alkyl; $C_{1-4}$ alkoxy; $C_{1-4}$ haloalkyl; nitro or cyano.

5. A compound according to claim 1, wherein $R^1$ is 2-chloro-5-pyridyl.

6. An agrochemical composition comprising a compound according to any of claims 1 to 5 as active ingredient, and a solid or liquid carrier therefor.

7. A composition according to claim 6, wherein the said solid carrier is a natural clay, silica or talc.

8. A composition according to claim 6, which contains in addition a wetting agent.

9. A composition according to claim 6 which is in the form of an aqueous emulsion of a solution of said active ingredient in an organic solvent therefor.

10. A composition according to claim 1 which is in the form of an aerosol.

11. A composition according to claim 10, which contains in addition one or more other agrochemicals.

12. A method of combating fungi on plants, which comprises applying to the growing plant, or the locus thereof, a fungicidally effective amount of a compound according to any of claims 1 to 5, or a composition according to any of claims 6 to 11.

13. A method of treating seeds, which comprises applying to the seeds a composition according to any of claims 1 to 5, or a composition according to any of claims 6 to 11.

14. A process of preparing a compound according to claim 1 which comprises reacting an acid chloride $R^1COCl$ wich amino-acetonitrile to obtain the amino-acetonitrile derivative $R^1CONHCH_2CN$, and reacting the said aminoacetonitrile derivative with a brominating agent to provide the corresponding brominated derivative

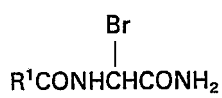

as first intermediate, reacting the said first intermediate with an alcohol or thiol $R^2XH$ to obtain the carbamoyl compound

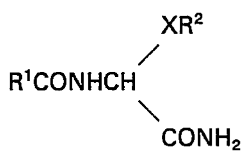

as second intermediate, and treating the said second intermediate with a dehydrating agent to convert it into the corresponding cyano compound, $R^1$ and X having the significance set forth in claim 1.

15. A process of preparing a compound according to claim 1, which comprises condensing an amide $R^1CONH_2$ with a glyoxylic ester HCO—$CO_2R^6$ to produce the hydroxy intermediate $R^1CONH$—$CH(OH)CO_2R^6$, treating the said hydroxy intermediate with a chlorinating agent to the corresponding chloro-derivative $R^1CONHCH(Cl)CO_2R^6$, which chloro-derivative is reacted with an alcohol, or thiol to produce the ester

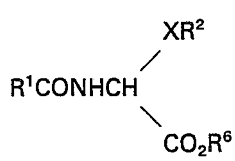

the said ester being converted by the action of ammonia to provide the carbamoyl compound

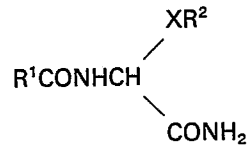

as second intermediate, and treating the said second intermediate to convert it into the corresponding cyano compound, $R^1$, $R^2$ and X having the significance set forth in claim 1, and $R^6$ is an ester radical.

16. A modification of the process of claim 15, wherein the said hydroxy intermediate is converted to the said ester in a single step by reaction with an alcohol $R^2OH$ in the presence of sulphuric acid as catalyst.

17. A process of preparing a compound according to claim 1, which comprises treating an amide $R^1CONH_2$ with sodium hydride and reacting the anion thus generated with an alpha bromo ester $R^2X{-}CH(Br)CO_2R^6$ to provide an amide ester.

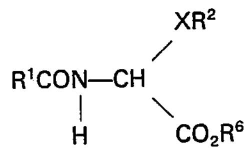

the said amide ester being subsequently reacted with ammonia to provide the corresponding amide

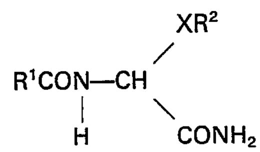

which amide is treated with a dehydrating agent to provide the corresponding nitrile, and wherein $R^1$, X and $R^6$ having the significance set forth in claim 1, and $R^6$ is an ester radical.

18. A process of preparing a compound according to claim 1, wherein an acylaminoacetonitrile $R^1CONHCH_2CN$ is reacted with a chlorinating or brominating agent in a solvent to provide a chloro- or bromo- derivative

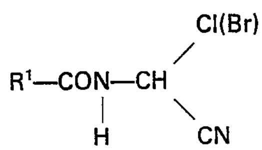

said chloro- or bromo- derivative being treated with an excess of a reactive alcohol or thiol in the presence of a base to convert it into the corresponding nitrile compound and wherein $R^1$ having the significance set forth in claim 1.

19. A process of preparing a compound according to claim 1, wherein gaseous $H_2S$ is passed through a solution of the nitrile

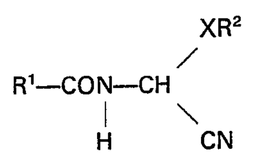

to produce the compound

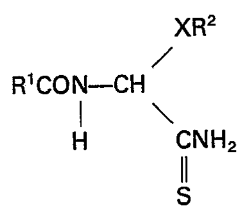

where $R^1$ and $R^2$ have the significance set forth in claim 1.

**Claims for the Contracting State: AT**

1. A process of preparing a compound of the general formula:

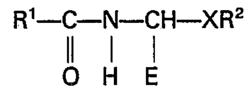

or a tautomer thereof, wherein:

$R^1$ is an aryl or substituted aryl radical, or a heterocyclyl or substituted heterocycyclyl radical;

X is oxygen or sulphur, and

E is CN or $CONH_2$; characterised in that $R^2$ is a $C_3$ to $C_7$ alkynyl group optionally substitued by $C_{1-4}$ alkyl, chlorine, bromine or iodine;

which comprises reacting an acid chloride $R^1COCl$ with amino-acetonitrile to obtain the amino-acetonitrile derivative $R^1CONHCH_2CN$, and reacting the said aminoacetonitrile derivative with a brominating agent to provide the corresponding brominated derivative

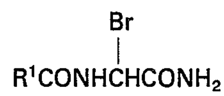

as first intermediate, reacting the said first intermediate with an alcohol or thiol $R^2XH$ to obtain the carbamoyl compound

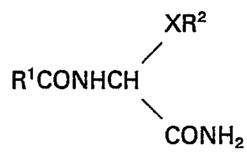

as second intermediate, and treating the said second intermediate with a dehydrating agent to convert it into the corresponding cyano compound.

2. A process of preparing a compound according to claim 1, which comprises condensing an amide $R^1CONH_2$ with a glyoxylic ester $HCO—CO_2R^6$ to produce the hydroxy intermediate $R^1CONH—CH(OH)CO_2R^6$, treating the said hydroxy intermediate with a chlorinating agent to form the corresponding chloro-derivative $R^1CONHCH(Cl)CO_2R^6$, which chloro-derivative is reacted with an alcohol, or thiol to produce the ester

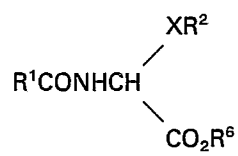

the said ester being converted by the action of ammonia to provide the carbamoyl compound

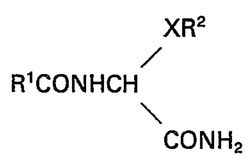

as second intermediate, and treating the said second intermediate to convert it into the corresponding cyano compound, $R^1$, $R^2$ and X having the significance set forth in claim 1, and $R^6$ being an ester radical.

3. A modification of the process of claim 2, wherein the said hydroxy intermediate is converted to the said ester in a single step by reaction with an alcohol $R^2OH$ in the presence of sulphuric acid as catalyst.

4. A process of preparing a compound according to claim 1, which comprises treating an amide $R^1CONH_2$ with sodium hydride and reacting the anion thus generated with an alpha bromo ester $R^2X—CH(Br)CO_2R^6$ to provide an amide ester

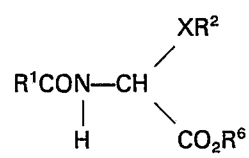

the said amide ester being subsequently reacted with ammonia to provide the corresponding amide

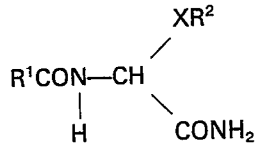

which amide is treated with a dehydrating agent to provide the corresponding nitrile, and wherein $R^1$, X and $R^6$ have the significance set forth in claim 1, and $R^6$ is an ester radical.

5. A process of preparing a compound according to claim 1, wherein an acylaminoacetonitrile $R^1CONHCH_2CN$ is reacted with a chlorinating or brominating agent in a solvent to provide a chloro- or bromo- derivative

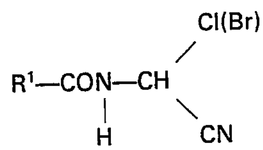

said chloro- or bromo- derivative being treated with an excess of a reactive alcohol or thiol in the presence of a base to convert it into the corresponding nitrile compound and wherein $R^1$ has the significance set forth in claim 1.

6. A process of preparing a compound according to claim 1, wherein $R^1$, X and $R^2$ are as defined and E is —$CSNH_2$, wherein gaseous $H_2S$ is passed through a solution of the nitrile

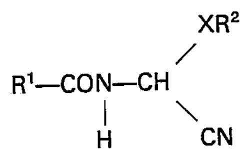

to produce the compound

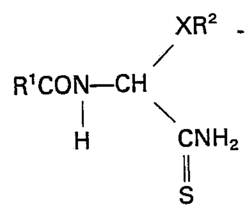

**Patentansprüche für die Vertragsstaaten: BE CH DE FR GB IT LI NL**

1. Verbindung der allgemeinen Formel

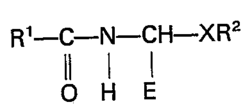

oder ein Tautomer derselben, worin

$R^1$ für einen Aryl- oder substituierten Arylrest oder einen heterocyclischen oder substituierten heterocyclischen Rest steht,

X für Sauerstoff oder Schwefel steht, und

E für CN, $CONH_2$ oder $CSNH_2$ steht,

dadurch gekennzeichnet, daß $R^2$ für eine $C_3$- bis $C_7$- Alkinylgruppe, die gegebenenfalls durch $C_{1-4}$-Alkyl, Chlor, Brom oder Jod substituiert ist, steht.

2. Verbindung nach Anspruch 1, in der $R^1$ für Phenyl oder substituiertes Phenyl steht.

3. Verbindung nach Anspruch 1, in der $R^1$ für Naphthyl oder substituiertes Naphthyl steht.

4. Verbindung nach Anspruch 1, in der $R^1$ für einen Aryl- oder heterocyclischen Rest, substituiert mit einer oder mehreren Gruppen, steht, wobei die substituierte Gruppe oder Gruppen Chlor, Brom oder Fluor, $C_{1-4}$-Alkyl, $C_{1-4}$-Alkoxy, $C_{1-4}$-Halogenalkyl, Nitro oder Cyano sein können.

5. Verbindung nach Anspruch 1, in der $R^1$ für 2-Chloro-5-pyridyl steht.

6. Agrochemische Zusammensetzung, die eine Verbindung nach einem der Ansprüche 1 bis 5 als aktive Komponente und einen festen oder flüssigen Trägerstoff hierfür aufweist.

7. Zusammensetzung nach Anspruch 6, in der der feste Trägerstoff ein natürlicher Ton, Siliciumdioxid oder Talk ist.

8. Zusammensetzung nach Anspruch 6, die zusätzlich ein Netzmittel enthält.

9. Zusammensetzung nach Anspruch 6, die in Form einer wäßrigen Emulsion einer Lösung der aktiven Komponente in einem organischen Lösungsmittel hierfür vorliegt.

10. Zusammensetzung nach Anspruch 6, die in Form eines Aerosols vorliegt.

11. Zusammensetzung nach Anspruch 10, die zusätzlich einen oder mehrere andere agrochemische Stoffe enthält.

12. Verfahren zur Bekämpfung von Fungi auf Pflanzen, bei dem auf die wachsende Pflanze oder die Wuchsstelle derselben eine fungicidisch wirksame Menge einer Verbindung gemäß einem der Ansprüche 1 bis 5 oder eine Zusammensetzung gemäß einem der Ansprüche 6 bis 11 aufgebracht wird.

13. Verfahren zur Behandlung von Samen, bei dem auf die Samen eine Verbindung gemäß einem der Ansprüche 1 bis 5 oder eine Zusammensetzung gemäß einem der Ansprüche 6 bis 11 aufgebracht wird.

14. Verfahren zur Herstellung einer Verbindung gemäß Anspruch 1, bei dem ein Säurechlorid $R^1COCl$ mit Aminoacetonitril zur Bildung des Aminoacetonitril-Derivats $R^1CONHCH_2CN$ umgesetzt wird und dieses Aminoacetonitril-Derivat mit einem Bromierungsmittel zur Bildung des entsprechenden bromierten Derivats

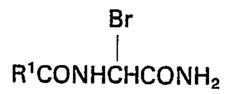

als erstes Zwischenprodukt umgesetzt wird, dieses erste Zwischenprodukt mit einem Alkohol oder Thiol $R^2XH$ zur Bildung der Carbamoylverbindung

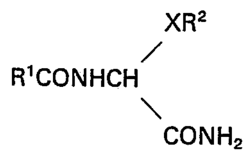

als zweites Zwischenprodukt umgesetzt wird, und dieses zweite Zwischenprodukt mit einem Dehydratisierungsmittel zur Umwandlung desselben in die entsprechende Cyanoverbindung behandelt wird, wobei $R^1$, $R^2$ und X die in Anspruch 1 angegebene Bedeutung haben.

15. Verfahren zur Herstellung einer Verbindung nach Anspruch 1, bei dem ein Amid $R^1CONH_2$ mit einem Glyoxylester $HCO—CO_2R^6$ zur Bildung des Hydroxy-Zwischenprodukts $R^1CONH—CH(OH)CO_2R^6$ kondensiert wird, dieses Hydroxy-Zwischenprodukt mit einem Chlorierungsmittel zur Bildung des entsprechenden Chlor-Derivats $R^1CONHCH(Cl)CO_2R^6$ behandelt wird, dieses Chlor-Derivat mit einem Alkohol oder Thiol zur Bildung des Esters

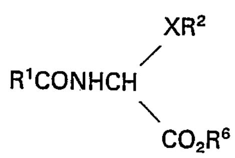

umgesetzt wird, dieser Ester durch Einwirkung von Ammoniak unter Bildung der Carbamoylverbindung

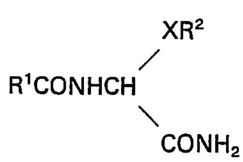

als zweites Zwischenprodukt umgewandelt wird, und dieses zweite Zwischenprodukt zur Umwandlung desselben in die entsprechende Cyanoverbindung behandelt wird, wobei $R^1$, $R^2$ und X die in Anspruch 1 angegebene Bedeutung haben und $R^6$ für einen Ester-Rest steht.

16. Abwandlung des Verfahrens nach Anspruch 15, bei dem das Hydroxy-Zwischenprodukt in den angegebenen Ester in einer einzigen Verfahrensstufe umgewandelt wird durch Umsetzung mit einem Alkohol $R^2OH$ in Gegenwart von Schwefelsäure als Katalysator.

17. Verfahren zur Herstellung einer Verbindung nach Anspruch 1, bei dem ein Amid $R^1CONH_2$ mit Natriumhydrid behandelt wird und das auf diese Weise erzeugte Anion mit einem alpha-Bromoester $R^2X—CH(Br)CO_2R^6$ zur Bildung eines Amidesters

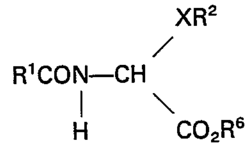

umgesetzt wird, dieser Amidester anschließend mit Ammoniak unter Bildung des entsprechenden Amids

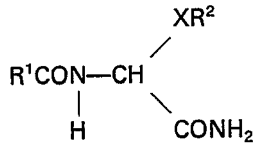

umgesetzt wird, und dieses Amid mit einem Dehydratisiermittel unter Bildung des entsprechenden Nitrils behandelt wird, wobei $R^1$, X und $R^2$ die in Anspruch 1 angegebene Bedeutung haben und $R^6$ für einen Ester-Rest steht.

18. Verfahren zur Herstellung einer Verbindung nach Anspruch 1, bei dem ein Acylaminoacetonitril $R^1CONHCH_2CN$ mit einem Chlorierungs- oder Bromierungsmittel in einem Lösungsmittel zur Bildung eines Chlor- oder Brom-Derivats

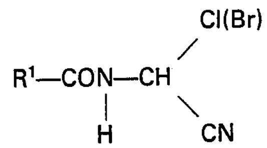

umgesetzt wird, und dieses Chlor- oder Brom-Derivat mit einem Überschuß an einem reaktiven Alkohol oder Thiol in Gegenwart einer Base zur Umwandlung desselben in die entsprechende Nitrilverbindung behandelt wird, wobei $R^1$ die in Anspruch 1 angegebene Bedeutung hat.

19. Verfahren zur Herstellung einer Verbindung nach Anspruch 1, bei dem gasförmiges $H_2S$ durch eine Lösung des Nitrile

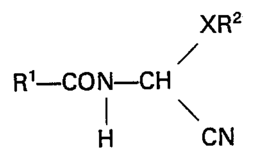

zur Bildung der Verbindung

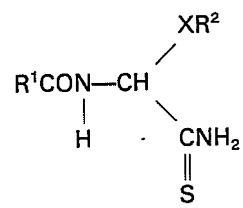

geleitet wird, wobei $R^1$ und $R^2$ die in Anspruch 1 angegebene Bedeutung haben.

**Patentansprüche für den Vertragsstaat: AT**

1. Verfahren zur Herstellung einer Verbindung der allgemeinen Formel

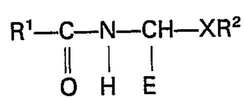

oder ein Tautomeren derselben, worin

$R^1$ für einen Aryl- oder substituierten Arylrest oder einen heterocyclischen oder substituierten heterocyclischen Rest steht,

X für Sauerstoff oder Schwefel steht, und

E für CN, $CONH_2$ oder $CSNH_2$ steht,

dadurch charakterisiert, daß $R^2$ für eine $C_3$- bis $C_7$- Alkinylgruppe, die gegebenenfalls durch $C_{1-4}$-Alkyl,

Chlor, Brom oder Jod substituiert ist, steht, bei dem ein Säurechlorid $R^1COCl$ mit Aminoacetonitril zur Bildung des Aminoacetonitril-Derivats $R^1CONHCH_2CN$ umgesetzt wird und dieses Aminoacetonitril-Derivat mit einem Bromierungsmittel zur Bildung des entsprechenden bromierten Derivats

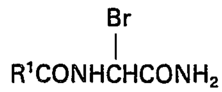

als erstes Zwischenprodukt umgesetzt wird, dieses erste Zwischenprodukt mit einem Alkohol oder Thiol $R^2XH$ zur Bildung der Carbamoylverbindung

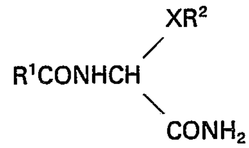

als zweites Zwischenprodukt umgesetzt wird, und dieses zweite Zwischenprodukt mit einem Dehydratisierungsmittel zur Umwandlung desselben in die entsprechende Cyanoverbindung behandelt wird.

2. Verfahren zur Herstellung einer Verbindung nach Anspruch 1, bei dem ein Amid $R^1CONH_2$ mit einem Glyoxylester $HCO—CO_2R^6$ zur Bildung des Hydroxy-Zwischenprodukts $R^1CONH—CH(OH)CO_2R^6$ kondensiert wird, dieses Hydroxy-Zwischenprodukt mit einem Chlorierungsmittel zur Bildung des entsprechenden Chlor-Derivats $R^1CONHCH(Cl)CO_2R^6$ behandelt wird, dieses Chlor-Derivat mit einem Alkohol oder Thiol zur Bildung des Esters

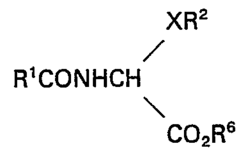

umgesetzt wird, dieser Ester durch Einwirkung von Ammoniak unter Bildung der Carbamoylverbindung

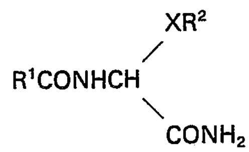

als zweites Zwischenprodukt umgewandelt wird, und dieses zweite Zwischenprodukt zur Umwandlung desselben in die entsprechende Cyanoverbindung behandelt wird, wobei $R^1$, $R^2$ und X die in Anspruch 1 angegebene Bedeutung haben und $R^6$ für einen Ester-Rest steht.

3. Abwandlung des Verfahrens nach Anspruch 2, bei dem das Hydroxy-Zwischenprodukt in den angegebenen Ester in einer einzigen Verfahrensstufe umgewandelt wird durch Umsetzung mit einem Alkohol $R^2OH$ in Gegenwart von Schwefelsäure als Katalysator.

4. Verfahren zur Herstellung einer Verbindung nach Anspruch 1, bei dem ein Amid $R^1CONH_2$ mit Natriumhydrid behandelt wird und das auf diese Weise erzeugte Anion mit einem alpha-Bromoester $R^2X—CH(Br)CO_2R^6$ zur Bildung eines Amidesters

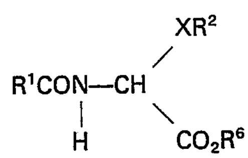

umgesetzt wird, dieser Amidester anschließend mit Ammoniak unter Bildung des entsprechenden Amids

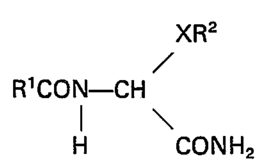

umgesetzt wird, und dieses Amid mit einem Dehydratisiermittel unter Bildung des entsprechenden Nitrils

behandelt wird, wobei $R^1$, X und $R^2$ die in Anspruch 1 angegebene Bedeutung haben und $R^6$ für einen Ester-Rest steht.

5. Verfahren zur Herstellung einer Verbindung nach Anspruch 1, bei dem ein Acylaminoacetonitril $R^1CONHCH_2CN$ mit einem Chlorierungs- oder Bromierungsmittel in einem Lösungsmittel zur Bildung eines Chlor- oder Brom-Derivats

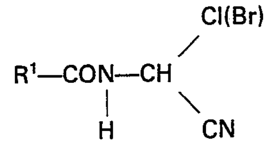

umgesetzt wird, und dieses Chlor- oder Brom-Derivat mit einem Überschuß an einem reaktiven Alkohol oder Thiol in Gegenwart einer Base zur Umwandlung desselben in die entsprechende Nitrilverbindung behandelt wird, wobei $R^1$ die in Anspruch 1 angegebene Bedeutung hat.

6. Verfahren zur Herstellung einer Verbindung nach Anspruch 1, wobei $R^1$, X und $R^2$ die angegebene Bedeutung haben und E für $-CSNH_2$ steht, bei dem gasförmiges $H_2S$ durch eine Lösung des Nitrils

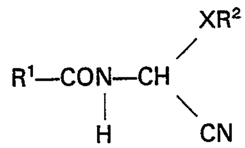

zur Bildung der Verbindung

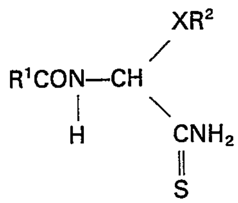

geleitet wird.

**Revendications pour les Etats contractants: BE CH DE FR GB IT LI NL**

1. Composé de formule générale:

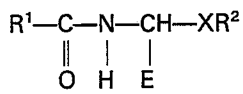

ou une de ses formes tautomères, formule dans laquelle:

$R^1$ est un radical aryle ou aryle substitué, ou un radical hétérocyclyle ou hétérocyclyle substitué;

X est l'oxygène ou le soufre, et

E est un groupe CN, $CONH_2$ ou $CSNH_2$; caractérisé en ce que $R^2$ est un groupe alcynyle en $C_3$ à $C_7$, facultativement substitué avec un groupe alkyle en $C_1$ à $C_4$, le chlore, le brome ou l'iode.

2. Composé suivant la revendication 1, dans lequel $R^1$ est un groupe phényle ou phényle substitué.

3. Composé suivant la revendication 1, dans lequel $R^1$ est un groupe naphtyle ou naphtyle substitué.

4. Composé suivant la revendication 1, dans lequel $R^1$ est un radical aryle ou hétérocyclyle substitué avec un ou plusieurs groupes, le ou les groupes substituants pouvant être le chlore, le brome ou le fluor; un groupe alkyle en $C_1$ à $C_4$; alkoxy en $C_1$ à $C_4$; halogénalkyle en $C_1$ à $C_4$, nitro ou cyano.

5. Composé suivant la revendication 1, dans lequel $R^1$ est un groupe 2-chloro-5-pyridyle.

6. Composition agrochimique comprenant un composé suivant l'une quelconque des revendications 1 à 5 comme ingrédient actif, et un support solide ou liquide destiné à cet effet.

7. Composé suivant la revendication 6, dans laquelle le support solide est une argile naturelle, la silice ou le talc.

8. Composition suivant la revendication 6, qui contient en outre un agent mouillant.

9. Composition suivant la revendication 6, qui est sous forme d'une émulsion aqueuse d'une solution de l'ingrédient actif dans un solvant organique de cet ingrédient.

10. Composition suivant la revendication 6, qui est sous forme d'un aérosol.

11. Composition suivant la revendication 10, qui contient en outre une ou plusieurs substances agrochimiques.

12. Procédé de lutte contre des champignons présents sur des plantes, qui consiste à appliquer à la plante en croissance, ou à son milieu, une quantité à effet fongicide d'un composé suivant l'une quelconque des revendications 1 à 5, ou d'une composition suivant l'une quelconque des revendications 6 à 11.

13. Procédé de traitement de graines, qui consiste à appliquer aux graines un composé suivant l'une quelconque des revendications 1 à 5, ou une composition suivant l'une quelconque des revendications 6 à 11.

14. Procédé de préparation d'un composé suivant la revendication 1, qui consiste à faire réagir un chlorure d'acide $R^1COCl$ avec de l'amino-acétonitile pour obtenir le dérivé d'amino-acétonitrile $R^1CONHCH_2CN$, et à faire réagir ledit dérivé d'amino-acétonitrile avec un agent de bromation pour donner le dérivé bromé correspondant

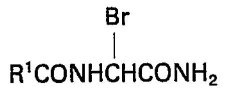

comme premier intermédiaire, à faire réagir le premier intermédiaire avec un alcool ou thiol $R^2XH$ pour obtenir le composé carbamoylé

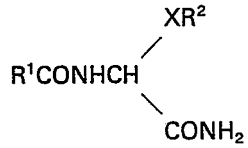

comme second intermédiaire, et à traiter le second intermédiaire avec un agent déshydratant pour le transformer en le composé cyané correspondant, $R^1$, $R^2$ et X ayant les significations indiquées dans la revendication 1.

15. Procédé de préparation d'un composé suivant la revendication 1, qui consiste à condenser un amide $R^1CONH_2$ avec un ester glyoxylique $HCO—CO_2R^6$ pour produire l'intermédiaire hydroxylé $R^1CONH—CH(OR)CO_2R^6$, à traiter l'intermédiaire hydroxylé avec un agent de chloration pour former le dérivé chloré correspondant $R^1CONHCH(Cl)CO_2R^6$, dérivé chloré qui est amené à réagir avec un alcool ou thiol pour produite l'ester

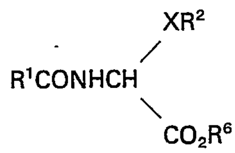

ledit ester étant transformé par l'action de l'ammoniac pour obtenir le composé carbamoylé

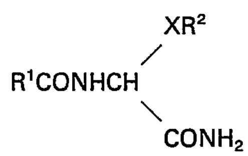

comme second intermédiaire, et à traiter le second intermédiaire pour le transformer en le composé cyané correspondant, $R^1$, $R^2$ et X ayant les significations indiquées dans la revendication 1, et $R^6$ étant un radical ester.

16. Modification du procédé suivant la revendication 15, dans laquelle l'intermédiaire hydroxylé est transformé en l'ester en une seule étape par réaction avec un alcool $R^2OH$ en présence d'acide sulfurique comme catalyseur.

17. Procédé de préparation d'une composé suivant la revendication 1, qui consiste à traiter un amide $R^1COHN_2$ avec de l'hydrure de sodium et à faire réagir l'anion ainsi engendré avec un ester alpha-bromé $R^2X—CH(Br)CO_2R^6$ pour obtenir un ester d'amide

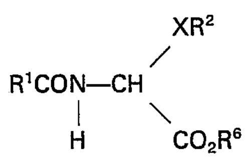

ledit ester d'amide étant ensuite amené à réagir avec l'ammoniac pour donner l'amide correspondant

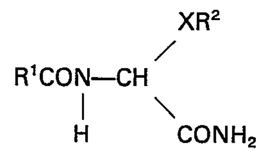

amide qui est traité avec un agent déshydratant pour donner le nitrile correspondant, et dans lequel $R^1$, X et $R^2$ ont les significations indiquées dans la revendication 1, et $R^6$ est un radical ester.

18. Procédé de préparation d'un composé suivant la revendication 1, dans lequel un acylamino-acétonitrile $R^1CONHCH_2CN$ est amené à réagir avec un agent de chloration ou de bromation dans un solvant pour donner un dérivé chloré ou bromé

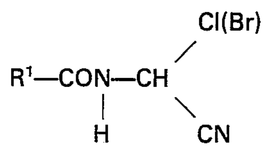

ledit dérivé chloré ou bromé étant traité avec un excès d'un alcool ou thiol réactif en présence d'une base pour le transformer en le nitrile correspondant, et dans lequel $R^1$ possède la signification indiquée dans la revendication 1.

19. Procédé de préparation d'un composé suivant la revendication 1, dans lequel $H_2S$ gazeux est passé à travers une solution du nitrile

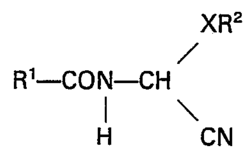

pour produire le composé

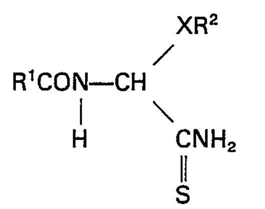

dans lequel $R^1$ et $R^2$ ont les significations indiquées dans la revendication 1.

**Revendications pour l'Etat contractant: AT**

1. Procédé de préparation d'un composé de formule générale:

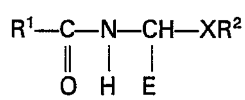

ou d'une de ses formes tautomères, formule dans laquelle:

$R^1$ est un radical aryle ou aryle substitué, ou un radical hétérocyclyle ou hétérocyclyle substitué;

X est l'oxygène ou le soufre, et

E est un groupe CN ou $CONH_2$; caractérisé en ce que $R^2$ est un groupe alcynyle en $C_3$ à $C_7$, facultativement substitué avec un groupe alkyle en $C_1$ à $C_4$, le chlore, le brome ou l'iode;

qui consiste à faire réagir un chlorure d'acide $R^1COCl$ avec l'amino-acétonitrile pour obtenir le dérivé d'amino-acétonitrile $R^1CONHCH_2CN$, et à faire réagir ledit dérivé d'amino-acétonitrile avec un agent de bromation pour donner le dérivé bromé correspondant

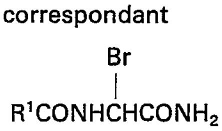

comme premier intermédiaire, à faire réagir ledit premier intermédiaire avec un alcool ou thiol $R^2XH$ pour obtenir le composé carbamoylé

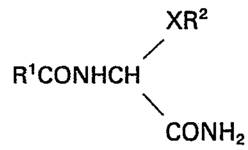

comme second intermédiaire, et à traiter ledit second intermédiaire avec un agent déshydratant pour le transformer en le composé cyané correspondant.

2. Procédé de préparation d'un composé suivant la revendication 1, qui consiste à condenser un amide $R^1CONH_2$ avec un ester glyoxylique $HCO—CO_2R^6$ pour produire l'intermédiaire hydroxylé $R^1COHN—CH(OH)CO_2R^6$, à traiter ledit intermédiaire hydroxylé avec un agent de chloration pour former le dérivé chloré correspondant $R^1CONHCH(Cl)CO_2R^6$, dérivé chloré qui est amené a réagir avec un alcool ou thiol pour produire l'ester

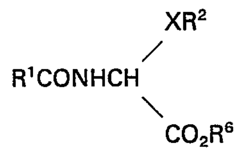

ledit ester étant transformé par l'action de l'ammoniac pour donner le composé carbamoylé

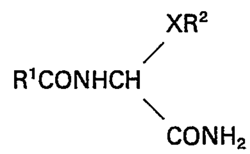

comme second intermédiaire, et à traiter ledit second intermédiaire pour le transformer en le composé cyané correspondant, $R^1$, $R^2$ et X ayant les significations indiquées dans la revendication 1, et $R^6$ étant un radical ester.

3. Modification du procédé suivant la revendication 2, dans laquelle l'intermédiaire hydroxylé est transformé en l'ester en une seule étape par réaction avec un alcool $R^2OH$ en présence d'acide sulfurique comme catalyseur.

4. Procédé de préparation d'une composé suivant la revendication 1, qui consiste à traiter un amide $R^1CONH_2$ avec de l'hydrure de sodium et à faire réagir l'anion ainsi engendré avec un ester alpha-bromé $R^2X—CH(Br)CO_2R^6$ pour donner un ester d'amide

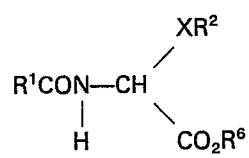

ledit ester d'amide étant ensuite amené à réagir avec l'ammoniac pour donner l'amide correspondant

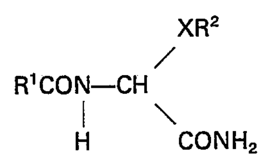

qui est traité avec un agent déshydratant pour donner le nitrile correspondant, et dans lequel $R^1$, X et $R^6$ ont les significations indiquées dans la revendication 1, et $R^6$ est un radical ester.

5. Procédé pour la préparation d'un composé suivant la revendication 1, dans lequel un acylamino-acétonitrile $R^1CONHCH_2CN$ est amené à réagir avec un agent de chloration ou de bromation dans un solvant pour donner un dérivé chloré ou bromé

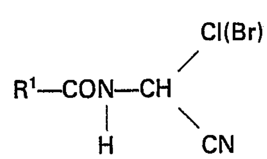

ledit dérivé chloré ou bromé étant traité avec un excès d'un alcool ou thiol réactif en présence d'une base

pour le transformer en le nitrile correspondant, et dans lequel $R^1$ a la signification indiquée dans la revendication 1.

6. Procédé de préparation d'un composé suivant la revendication 1, dans lequel $R^1$, X et $R^2$ répondent aux définitions mentionnées et E est un groupe $—CSNH_2$, dans lequel $H_2S$ gazeux est passé à travers une solution du nitrile

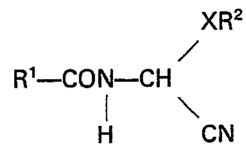

pour produire le composé